# EUROPEAN PATENT SPECIFICATION

(11) **EP 2 258 337 B1**
(45) Date of publication and mention of the grant of the patent: **20.05.2015**
(21) Application number: 09007422.0
(22) Date of filing: 03.06.2009
(51) Int. Cl.: A61K 8/362, A61K 8/49, A61Q 5/02, A61Q 5/12, A61K 8/365

(54) **Composition for hair**
Haarpflegezusammensetzung
Composition pour cheveux

(43) Date of publication of application: 08.12.2010
(73) Proprietor: Kao Germany GmbH, 64297 Darmstadt (DE)
(72) Inventor: Molenda, Michael, 60318 Frankfurt (DE); Tietjen, Ilka, 68549 Ilvesheim (DE); Herzog-Renker, Beate, 64291 Darmstadt (DE); Hermes, Frank, 64665 Alsbach-Hähnlein (DE)
(74) Representative: Grit, Mustafa

(56) References cited:
- WO-A-2004/105705
- WO-A-2008/027541
- WO-A-2008/054609
- DE-A1-102006 050 398
- US-A- 5 523 090
- US-A- 5 785 962

## Description

Present invention relates to a composition for hair comprising at least one xanthine or its derivative and at least one carboxylic acid according to the chemical structure given in the description below. Compositions of the present invention gives hair improved rigidity, grip, less elasticity and volume, but more body.

Conditioning compositions of various kinds have been known in the cosmetic art for a long time. It is customary that such kind of compositions comprise conditioning ingredients mainly of cationic character and makes hair soft and smooth gives hair combability but hair loses its rigidity so that it does not hold its style for a long time and moves unwillingly with any movement of head of person. People especially with fine hair and also with damaged hair used to apply such kind of conditioners in order to make hair combable, but hair becomes at the same time soft and smooth and therewith loses its rigidity.

US 2006/0039878 A1 discloses hair treatment compositions comprising α -hydroxy acid and a xanthine derivative for styling and lengthening hair which furthermore reduces the frizzes and increase hair volume. The α-hydroxy acids especially mentioned and claimed are citric and tartaric acids.

Inventors of the present invention have surprisingly found out that a composition comprising at least one xanthine or its derivatives and a carboxylic acid other than an α-hydroxy acid makes hair synergistically more rigid, less smooth compared to a composition comprising the ingredients individually, reduces its volume but increases its body.

Accordingly the first object of the present invention is a conditioning composition for hair comprising at least one xanthine or its derivative according to the general formula 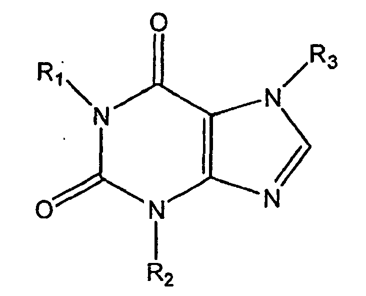 wherein R₁, R₂ and R₃ are independent from each other H, or substituted or unsubstituted C₁ to C₅ alkyl, wherein independent from each other R₅ at least one carboxylic acid selected from malonic acid and glyoxylic acid and at least one conditioning compound selected from oily substances, nonionic substances, cationic amphiphilic ingredients, cationic polymers or their mixture.

Further object of the present invention is the use of the composition for conditioning hair and especially for improving rigidity and body of hair.

WO 2004/105705 A1 discloses xanthine-comprising compositions. However, the document fails to guide and disclose xanthine in combination with the claimed carboxylic acids and conditioner within the meaning of the present invention.

Compositions of the present invention are preferably aqueous compositions and comprise at least 10% by weight water, calculated to total composition. More preferably the water concentration varies between 20 and 90% by weight calculated to total composition.

Conditioning composition of the present invention can be in any preparation form suitable for application onto hair and preferably is in the form of solution, dispersion, gel, emulsion, spray and foam.

Composition of the present invention comprises at least one xanthine or its derivative according to the formula given above preferably at a concentration of 0.001 to 5%, more preferably 0.005 to 3% and most preferably 0.01 to 2.5% and particularly preferably 0.05 to 1.5% by weight calculated to total composition. In a further preferred embodiment of the present invention, R₁, R₂ and R₃ are independent from each other H or CH₃ and more preferably at least 1 of them is CH₃, most preferably at least 2 of them are CH₃ and particularly preferably all of them are CH₃. Accordingly the preferred xanthines and its derivatives are xanthine, caffeine, theophylline and theobromine, and more preferred are caffeine, theophylline and theobromine. Particularly preferred is caffeine.

Compositions of the present invention comprise at least one carboxylic acid at a concentration of 0.001 to 5%, more preferably 0.005 to 3% and most preferably 0.01 to 2.5% and particularly preferably 0.05 to 1.5% by weight calculated to total composition. Particularly preferred is glyoxylic acid.

In a further preferred embodiment, it has been found out that the weight ratio of the xanthine and its derivative according to the formula above to carboxylic acid according to the formula above plays a role in achieving the optimum performance of the compositions and therefore both components are present at a weight ratio of 10:1 to 1:10, preferably 5:1 to 1:5, more preferably 2:1 to 1:2 and most preferably 1:1.

Compositions of the present invention can be either a conditioning -cleansing composition or a conditioning composition typically used after use of cleansing compositions

Composition of the present invention comprises hair-conditioning agents in any type of composition. Conditioning agents are selected from oily substances, non-ionic substances, cationic amphiphilic ingredients, cationic polymers or their mixtures.

Oily substances are selected from such as volatile or non-volatile silicone oils, natural oils other than falling in the definition of claim 1 and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule, natural oils such as olive oil, almond oil, avocado oil, ricinus oil, Passiflora oil (Passiflora edulis, Passiflora incarnate), Black cumin oil (Nigella sativa), Borage oils (Borage officinalis), Evening Primrose oil (Oenotera biensis), Grapeseed oil (Vitis vinifera), Hempseed oil (Cannabis sativa), Kukui nut oil (Aleurites moluccans), Rosehip oil (Rosa moschata), Safflower oil (Carthamus tinctorius), Walnut oil (Juglans nigra) and Wheatgerm oil (Triticum vulgare), and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyethyleneglycol mono or di fatty acid esters having general formula

R₇CO(OCH₂CH₂)ₙOH

or

R₇CO(OCH₂CH₂)ₙOOCR₈

wherein R₇ and R₈ are independent from each other saturated, unsaturated or branched or non-branched alkyl chain with 7 to 21 C atoms and n is typically 2 - 100.

In another preferred from of the present invention, compositions comprise at least one cationic conditioning agent. Cationic conditioning agents are cationic polymers and cationic or cationizable surfactants. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose and its derivatives, cationic guar gum and its derivatives, cationic Caesalpinia spinosa gum and its derivatives, polyquaternium 6, polyquaternium 7, polyquaternium 67, polyquaternium 70, and polyquaternium 87.

The cationic polymers also include the quaternized products of graft polymers from organopolysiloxanes and polyethyl oxazolines described in EP-A 524 612 and EP-A 640 643.

Cationic and/or cationizable surfactant compounds according to the present invention are preferably of the general structure

R₉-A-R₁₀-B

wherein R₉ is a saturated or unsaturated, straight or branched alkyl group with 8 to 24 C atoms, R₁₀ is a straight or branched alkyl group with 1 to 4 C atoms, A is a group selected from O, 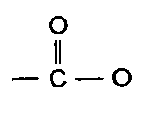 and 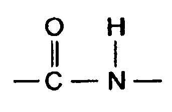 and B is selected from 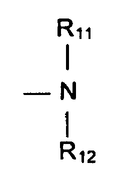 wherein R₁₁ and R₁₂ are the same or different is H or an alkyl with 1 to 4 C atoms, hydroxyl alkyl with 1 to 4 C atoms and di hydroxyl alkyl with 2 to 4 C atoms, 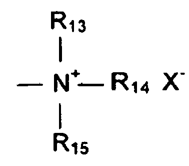 R₁₃ and R₁₅ are the same or different, an alkyl with 1 to 4 C atoms, hydroxyl alkyl with 1 to 4 C atoms and di hydroxyl alkyl with 2 to 4 C atoms, R₁₄ is an alkyl with 1 to 4 C atoms, hydroxyl alkyl with 1 to 4 C atoms or di hydroxyl alkyl with 2 to 4 C atoms and

-R₁₀-A-R₉

wherein R₉, A and R₁₀ have the above meaning and X is chloride, bromide, methosulfate,
or
a quaternary ammonium surfactant according to the general formula 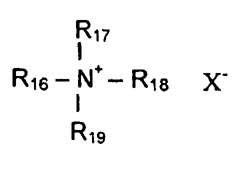 where R₁₆ is a saturated or unsaturated, branched or non-branched alkyl chain with 8-24 C atoms and R₁₇ is unsaturated or saturated, branched or non-branched alkyl chain with 1 - 24 C atoms and R₁₈ and R₁₉ are lower alkyl chain with 1 to 4 carbon atoms which may be substituted with one or more hydroxyl groups, and X is anion such as chloride, bromide, methosulfate.

Compositions of the present invention comprise at least one cationic and/or cationizable surfactant compound according to the above general structures. In the preferred embodiment of the present invention, R₁ is saturated or unsaturated, straight or branched alkyl group with 10 to 24C atoms, more preferably 12 to 22 C atoms and R₂ is straight or branched alkyl group with 1 to 4 C atoms, A, B, R₃ to R₇ are same as above. Non-limiting suitable examples are stearyloxypropyl amine, palmityloxypropyl amine, stearyloxypropyldimethyl amine, stearyloxypropyldiethyl amine, stearyloxyethylyldimethyl amine, stearyloxyethyl amine, myristyloxypropyl amine, myristyloxypropyldimethyl amine, palmitamidopropyl amine, palmitamidopropyl methylamine, palmitamidopropyl diethylamine, palmitamidopropyl dibutylamine, palmitamidopropyl buylamine, palmitamidopropyl dipropylamine, palmitamidopropyl propylamine, palmitamidopropyl dihydroxyethylamine, palmitamidopropyl hydroxyethylamine, palmitamidopropyl dihydroxypropylamine, palmitamidopropyl hydroxypropylamine, lauramidopropyl amine, lauramidopropyl methylamine, lauramidopropyl diethylamine, lauramidopropyl dibutylamine, lauramidopropyl buylamine, lauramidopropyl dipropylamine, lauramidopropyl propylamine, lauramidopropyl dihydroxyethylamine, lauramidopropyl hydroxyethylamine, lauramidopropyl dihydroxypropylamine, lauramidopropyl hydroxypropylamine, stearamidopropyl amine, stearamidopropyl methylamine, stearamidopropyl diethylamine, stearamidopropyl dibutylamine, stearamidopropyl butylamine, stearamidopropyl dipropylamine, behenamidopropyl propylamine, behenamidopropyl dihydroxyethylamine, behenamidopropyl hydroxyethylamine, behenamidopropyl dihydroxypropylamine, behenamidopropyl hydroxypropylamine, behenamidopropyl amine, behenamidopropyl methylamine, behenamidopropyl diethylamine, behenamidopropyl dibutylamine, behenamidopropyl butylamine, behenamidopropyl dipropylamine, behenamidopropyl propylamine, behenamidopropyl dihydroxyethylamine, behenamidopropyl hydroxyethylamine, behenamidopropyl dihydroxypropylamine, behenamidopropyl hydroxypropylamine, dipalmitamidopropyl methylamine, dipalmitamidopropyl ethylamine, dipalmitamidopropyl butylamine, dipalmitamidopropyl propylamine, dipalmitamidopropyl hydroxyethylamine, dipalmitamidopropyl hydroxypropylamine, dilauramidopropyl amine, dilauramidopropyl methylamine, dilauramidopropyl buylamine, dilauramidopropyl hydroxyethylamine, dilauramidopropyl hydroxypropylamine, distearamidopropyl amine, distearamidopropyl methylamine, dibehenamidopropyl propylamine, dibehenamidopropyl hydroxyethylamine, palmitoamidopropyl trimethyl ammonium chloride, stearamidopropyl trimethylammonium chloride, behenamidopropyl tri hydroxyethalmonium chloride, distearylamidopropyl dimethyl ammonium chloride, dicetylamidodihydroxyethyl ammonium chloride, palmitoylpropyl amine, palmitoylpropyl methylamine, palmitoylpropyl diethylamine, palmitoylpropyl dibutylamine, palmitoylpropyl buylamine, palmitoylpropyl dipropylamine, palmitoylpropyl propylamine, palmitoylpropyl dihydroxyethylamine, palmitoylpropyl hydroxyethylamine, palmitoylpropyl dihydroxypropylamine, palmitoylpropyl hydroxypropylamine, myristoylpropyl amine, myristoylpropyl methylamine, myristoylpropyl diethylamine, myristoylpropyl dibutylamine, myristoylpropyl buylamine, myristoylpropyl dipropylamine, myristoylpropyl propylamine, myristoylpropyl dihydroxyethylamine, myristoylpropyl hydroxyethylamine, myristoylpropyl dihydroxypropylamine, myristoylpropyl hydroxypropylamine, stearoylpropyl amine, stearoylpropyl methylamine, stearoylpropyl diethylamine, stearoylpropyl dibutylamine, stearoylpropyl butylamine, stearoylpropyl dipropylamine, behenylpropyl propylamine, behenylpropyl dihydroxyethylamine, behenylpropyl hydroxyethylamine, behenylpropyl dihydroxypropylamine, behenylpropyl hydroxypropylamine, behenylpropyl amine, behenylpropyl methylamine, behenylpropyl diethylamine, behenylpropyl dibutylamine, behenylpropyl butylamine, behenylpropyl dipropylamine, behenylpropyl propylamine, behenylpropyl dihydroxyethylamine, behenylpropyl hydroxyethylamine, behenylpropyl dihydroxypropylamine, behenylpropyl hydroxypropylamine, dipalmitoylpropyl methylamine, dipalmitoylpropyl ethylamine, dipalmitylpropyl butylamine, dipalmitylpropyl propylamine, dipalmitylpropyl hydroxyethylamine, dipalmitylpropyl hydroxypropylamine, dilauroylpropyl amine, dilauroylpropyl methylamine, dilauroylpropyl buylamine, dilauroylpropyl hydroxyethylamine, dilauroylpropyl hydroxypropylamine, distearylpropyl amine, distearylpropyl methylamine, dibehenylpropyl propylamine, dibehenylpropyl hydroxyethylamine, palmitylpropyl trimethyl ammonium chloride, stearylpropyl trimethylammonium chloride, behenylpropyl tri hydroxyethalmonium chloride, distearylpropyl dimethyl ammonium chloride, dicetyldihydroxyethyl ammonium chloride, dioleoylethylhydroxyethylmonium methosulfate, dicocoylethylhydroxyethylmonium methosulfate, cetyltrimethyl ammonium chloride, steartrimonium chloride, behentrimonium chloride, myristyltrimethyl ammonium chloride, distearyldimethyl ammonium chloride, and dibehenyldimethyl ammonium chloride.

The compositions according to the invention may also comprise further conditioning substances such as protein hydrolyzates, polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}" and protein derivatives such as glycoproteins.

Typical concentration range for any of those conditioners of cationic polymers, silicon oil and derivatives and cationic and/or cationizable surfactants is in the range of 0.01 - 10% by weight, preferably 0.01 - 7.5% by weight, more preferably 0.05 - 5% and most preferably 0.1 - 5% by weight calculated to the total composition. It should be noted that especially non-cleansing conditioning type of products contain higher concentrations (of the above mentioned concentrations) of the cationic surfactants which at the same time, if desired, can be emulsifying agent. In cleansing and conditioning type of preparations, preferably the concentration of cationic surfactants is towards the lower end of the above mentioned concentration, approximately below 1% by weight.

Composition of the present invention comprises at least one UV filter selected from water soluble hydrophilic ones and oil soluble lipophilic ones. The most preferred are the lipohilic oil soluble ones. Suitable UV-absorbing substances is are ethylhexylmethoxycinnamate, 4-aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4.4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzylidene campher, 3-(4'-sulfo)-benzylidene bornane-2-one and the salts thereof and/or 3-(4'-methyl benzylidene)-DL-campher. Preferred are ethylhexylmethoxycinnamate, benzophenones especially 2.4-dihydroxybenzophenone, 2,2',4,4'-tetrahydroxy- benzophenone, 2-hydroxy-4-methoxybenzophenone, 2,2'-dihydroxy-4,4'-dimethoxybenzophenone, 2-hydroxy-5-chlorobenzophenone, 2,2'-dihydroxy-4-methoxybenzophenone, 2-hydroxy-4-octyloxybenzophenone, 2-hydroxy-4-methoxy-4'-methylbenzophenone, 3-benzylidene campher, 3-(4'-sulfo)-benzylidene bornane-2-one and the salts thereof and/or 3-(4'-methyl benzylidene)-DL-campher, Polysilicone-15. Most preferred are ethylhexylmethoxycinnamate, polysilicone-15, benzophenone-3. The amount of the UV-absorber ranges typically from about 0.01 % to 7.5%, more preferably from 0.05 % to 5 % by weight, calculated to the total composition.

In one of the preferred embodiment of the present invention, compositions additionally comprise at least one polyol. The term polyol refers to a compound carrying 2 or more hydroxyl groups in its molecule.

Non-limiting suitable examples to the polyols preferred within the scope of the present invention are ethylene glycol and its homo polymers with varying molecular weight known with their INCI names such as diethyleneglycol, triethylene glycol and PEG with 4 to 800 ethylene glycol units, propylene glycol and its homo polymers with varying molecular weight known with their INCI names such as dipropylene glycol, tripropylene glycol and PPG with 4 to 50 propylene glycol units and glycerin and its homo polymers known with their INCI names diglycerin, triglycerin and polyglycerin with 4 to 40 glycerin units. Any of the homopolymers mentioned here can be liquid, semisolid, or solid. In the preferred from of the invention suitable polyols are the ones either liquid or semiliquid at ambient temperature. The most preferred are liquid ones at ambient temperature such as propylenegylcol, dipropylene glycol, PPG-9, diethylene glycol, triethylene glycol and glycerin.

Concentration of one or more polyol in the composition is varying between 0.01 and 25%, preferably 0.1 and 20%, more preferably 0.25 and 15% and most preferably 0.25 and 10% by weight calculated to total composition. The concentration ranges mentioned here refer to the total concentration of polyols in case there are more than one polyol present in the compositions.

Compositions of the present invention are cleansing compositions (cleansing-conditioning composition). Cleansing compositions of the present invention comprise at least one surfactant selected from anionic, non-ionic and/or amphoteric or zwitterionic surfactants at a concentration range of 1 to 50%, preferably 5 to 40% and more preferably 5 to 30%, and most preferably 5 to 25% by weight, calculated to the total composition.

In one of the preferred embodiment of the present invention cleansing composition of the present invention, comprises at least one anionic, at least one nonionic surfactant. More preferably, the compositions further comprise additionally at least one amphoteric surfactant.

Anionic surfactants suitable within the scope of the invention are preferably present in an amount from 1 to about 30%, preferably 2 to 20% and most preferably 2 - 15%, by weight, calculated to the total composition.

These are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₂₀-(C₂H₄O)ₙ-O-CH₂COOX,

wherein R₂₀ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 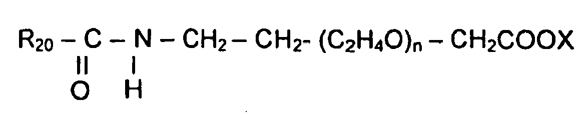 wherein R₂₀ and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

It is also possible to use mixtures of several anionic surfactants, for example an ether sulfate and a polyether carboxylic acid or alkyl amidoether carboxylic acid.

Further suitable anionic surfactants are also C₈-C₂₂-acyl aminocarboxylic acids or the water-soluble salts thereof. Especially preferred are N-lauroyl glutamate and Cocoyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine, N-lauroyl aminopropyl glycine, cocoyl taurate, cocoyl alaninate, lauroyl alaninate preferably in form of the water-soluble alkali or ammonium, in particular the sodium salts thereof, preferably in admixture with the above-named anionic surfactants.

Further surfactants in the shampoo compositions according to the invention are nonionic surfactants especially in admixture with anionic surfactants.

Especially suited are alkyl polyglucosides of the general formula

R₂₁-O-(R₂₂O)ₙ-Zₓ,

wherein R₂₁ is an alkyl group with 8 to 18 carbon atoms, R₂₂ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components are, for example, long-chain fatty acid mono- and dialkanolamides, such as coco fatty acid monoethanolamide and myristic fatty acid monoethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further nonionic surfactants useful in the compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Total concentration of nonionic surfactants in compositions of the present invention is in the range of 1 to 20%, preferably 1 to 15% and more preferably 1 to 10% by weight calculated to total composition.

As further surfactant component, in another preferred form of invention, the compositions especially those of cleansing and conditioning according to the invention comprise at least one amphoteric or zwitterionic surfactant in an amount from 0.5 % to 15 %, preferably from 1 % to 10 %, more preferably from 1 % to 7.5 %by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio is preferably be in the range of 10:1 to 1:1, more preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 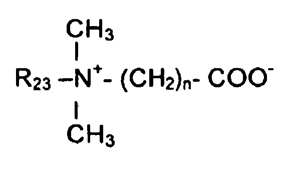 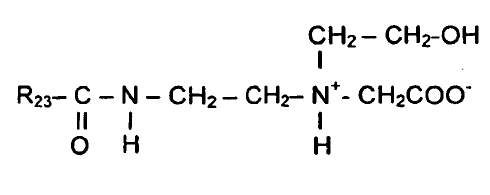 wherein R₂₃ is a C₈-C₁₈-alkyl group and n is 1 to 3;
sulfobetaines of the structure 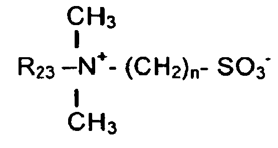 wherein R₂₃ and n are same as above;
and amidoalkyl betaines of the structure 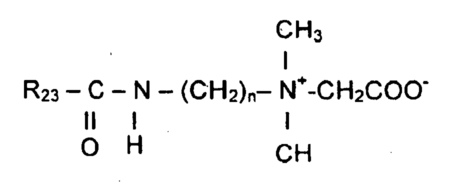 wherein R₂₃ and n are same as above.

In another preferred form of the invention, cleansing composition of the present invention comprise at least one anionic, at least one non-ionic and at least one amphoteric surfactants.

Solubilizers may be added to the compositions, in particular cleansing compositions, especially when oily substances are chosen as conditioning agents and fragrance oils with highly lipophilic properties. Typical solubilizers may be hydrogenated castor oil known with the trade mark Cremophor RH series from BASF. It should be noted that the surfactant mixture as well be a good solubilizer for fragrance oils. Typical concentration of the solubilizers can be in the range of 0.01 - 2% by weight, preferably 0.1 - 1% by weight, calculated to total composition.

Further conditioning additives are hair conditioning and/or styling polymers into either cleansing or conditioning type compositions. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning composition of any type of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryl oylethyl betaine and alkyl - methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl - aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Conditioning and cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Hair cleansing conditioning compositions of the present invention can be in the form of conventional liquid thickened shampoo, as well in the form of ready to use foam, delivered either from a pump-foamer or from an aerosol bottle. In the case that an aerosol foam preparation is preferred, propellant gas must be added to the formulation. The suitable propellant gasses are carbondioxide, dimethylether and alkanes such as butane propane or their mixtures.

Compositions of the present invention used after cleansing hair can be in the form of emulsions, solutions, gels and dispersions. In the case that solutions and/or gels forms are preferred the appearance can be either with a transparent or opaque. As a product form, foam is as well suited when packed into a pressurized can or delivered through a pump-foamer (non-aerosol). In the case that an aerosol foam preparation is preferred, propellant gas must be added to the formulation. The suitable propellant gases are carbon dioxide, dimethylether and alkanes such as butane, propane, isobutane or their mixtures.

Without any limitation, preferably the emulsion type of conditioners comprise additionally at least one fatty alcohol of the following formula

R₁₄-OH

where R₁₄ is a saturated or unsaturated, branched or non-branched alkyl chain with 8 - 24 C atoms. Concentration of fatty alcohols is usually less than 20%, preferably less than 15%, more preferably in the range of 1 to 10% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

Emulsion type of conditioners comprise in addition to fatty alcohol at least one emulsifier. Suitable emulsifiers are especially non-ionic and cationic surfactants. They can certainly be present also in mixture. Among the non-ionic ones especially preferred are fatty alcohol ethoxylates as mentioned above. Cationic surfactants suitable are mono long chain alkyl carrying ones as disclosed above. Both surfactants are present in the compositions of the present invention in concentration ranges as disclosed above.

Composition comprises organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylene carbonate, ethylene glycol monoethylether, ethylene glycol monobutyl ether, ethylene glycol monophenyl ether, 1-phenylethylalcohol, 2-phenylethylalcohol, o-methoxyphenol. The most preferred ones are benzyl alcohol, ethanol, benzyloxyethanol, Concentration of organic solvents should not exceed 10% by weight, preferably in the range of 0.1 to 7.5% by weight calculated to total composition in compositions designed for cleansing and conditioner / treatment in emulsion form. Higher organic solvent concentrations may be suitable for the preparation such as hair conditioning solution applied by a spraying device. In such case organic solvent concentration can be as high as 50% by weight.

The compositions of present invention can comprise active ingredients such as sequestering agents, natural ingredients, flavonoids, ceramides, pyrrolicdone carboxylic acid or its salts and its esters with an alkyl chain etc.

The sequestering agents are selected from polycarboxy acids and their salts. The preferred ones are ethylene diamine tetraacetic acid (EDTA), ethylene diamine disuccinate and their salts. Typical useful concentration range for sequestering agents is of 0.01 - 2.5% by weight calculated to the total composition.

Natural plant extracts may be incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R} ", "Sedaplant^{R}", "Hexaplant^{R}" and "Crodarom^{R}". Extracts and the preparation thereof are also described in "Hagers Handbuch der pharmazeutischen Praxis", 4th Ed..

Compositions of the present invention optionally may comprise at least one flavone and/or its derivative at a concentration in the range of 0.01% to 2%, by weight, preferably 0.01 to 1.5% and more preferably 0.05 to 1.0% by weight, calculated to total composition.

Suitable non-limiting examples are t-flavanone, quercetin, morin, taxifoline, chatechin, epichatechin and luteolin, The most preferred ones are t-flavanone (trans 3,4'-dimethylflavanonol) , quercetin and morin. The flavone and its derivatives are incorporated into the compositions of the present invention either as commercially available pure (the word pure should not be taken as 100% purity and should be understood as flavone derivative enriched raw materials containing other substances) raw material and as well as in the form of natural extracts such as green tea extract, gingko extract, etc.

Composition of the present invention may optionally comprise at least one pyrrolidon carboxylic acid ester of the above formula. The alkyl chain length, in the preferred form, is between 8 and 20 C atoms, more preferably between 12 and 18 C atoms and most preferably between 14 and 18 C atoms.

Some examples to the suitable pyrrolidon carboxylic acid ester are with the alkyl chain of methyl, ethyl, n-propyl, isopropyl, n-butyl, iso butyl, n-hexyl, n-octyl, n-nonyl, n-decyl, ethylhexyl, methylhexyl, capryl, lauryl myristyl, cetyl, octyldodecyl, isostearyl, stearyl, arachidyl, behenyl, oleyl, linoleyl, benzyl, phenoxyethyl. Preferred are n-octyl, n-nonyl, n-decyl, ethylhexyl, methylhexyl, capryl, lauryl myristyl, cetyl, octyldodecyl, stearyl, isostearyl, arachidyl, behenyl, oleyl and linoleyl. More preferred are lauryl myristyl, cetyl, octyldodecyl, stearyl, isostearyl, arachidyl, behenyl, oleyl, and linoleyl. Particularly preferred pyrrolidon carboxylic acid ester is octyldodecyl pyrrolidon carboxylic acid which is available under the trade name Sebumol ODPC from Zschimmer & Schwarz.

Concentration of at least one pyrrolidon carboxylic acid ester in the compositions of the present invention is in the range of 0.01 to 5% by weight calculated to total composition. It should be noted that compositions of the present invention can comprise more than one pyrrolidon carboxylic acid ester such as 2 or 3. The above mentioned concentrations refer to the total concentration of the pyrrolidon carboxylic acid esters.

Composition of the present invention comprises pyrrolidon carboxylic acid and/or its salts. In principal any pyrrolidon carboxylic acid salt is suitable within the meaning of the present invention. Suitable examples are aluminium, calcium, copper, magnesium, potassium, sodium and zinc salts of pyrrolidon carboxylic acid. Preferred are aluminium, calcium, magnesium, potassium and sodium salts and more preferred are sodium and potassium salts. The most preferred is sodium salt.

Concentration of pyrrolidon carboxylic acid and/or its salts is in the range of 0.01 to 10% by weight calculated to the total composition. Concentrations mentioned here refer to the total concentration of the pyrrolidon carboxylic acid and/or its salts present in the compositions.

According to the preferred embodiment of the present invention, at least one pyrrolidone carboxylic acid ester of the above formula and pyrrolidone carboxylic acid and/or its salts are comprised in the composition of the present invention at a weight ratio of 1:100 to 1:1, preferably 1:75 to 1:2, more preferably 1:50 to 1:3 and most preferably 1:25 to 1:5.

The pH of the compositions according to the present invention is suitably between 2 and 8.0, preferably in the range of 2.5 to 7.0, more preferably 3 to 6.5 and most preferably 4 to 5.5 measured at ambient temperature with a suitable pH meter. pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Further in preferred embodiment of the present invention, compositions comprise at least one direct dye. Suitable direct dyes are of cationic, anionic and neutral nitro dyes. It should be noted that they can also be used in combination with each other. In other words, a composition according to present invention can comprise an anionic and a cationic dye as well as an anionic and a nitro dye or a cationic and a nitro dye. Certainly the combination of all three dyestuffs is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their salts

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 3-nitro-p-hydroxyethylaminophenol, 1-Hydroxy-2-amino-3-nitrobenzol and 2-hydroxyethylpicramic acid.

Concentration of one or more direct dyes in total is in the range of 0.001 to 5% by weight, preferably 0.01 to 4% more preferably 0.05 to 3% and most preferably 0.1 to 2.5% by weight calculated to total composition.

Cleansing compositions of the present invention can also comprise synthetic mica as a further shine enhancer. Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail, the content of the document is included herewith by reference. It also discloses a cleansing composition comprising monoethanolamide surfactant in addition to other surfactants.

Suitable metal oxide or oxides for coating synthetic mica are titanium dioxide, chromium oxide, ferric oxide or mixtures thereof. In the present invention the preferred is synthetic mice coated with titanium dioxide. Such materials are commercially available from Sun Chemical Corporation and known with their INCI names Synthetic Fluorphologopite.

The particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0.05 to 7.5%, more preferably 0.1 to 5% and most preferably 0.2 to 2.5% by weight calculated to total composition.

The viscosity of the conditioning shampoo compositions according to the invention is in the range of 500 and about 20,000 mPa.s at 20°C, preferably 1,000 to 10,000, in particular 1,500 to 8,000 mPa.s at 20°C, measured with Höppler viscosimeter.

Viscosity of shampoo compositions can be adjusted with known viscosity enhancers. The preferred ones are monoglycerides such as glyceryl laurate, oleate, PEG-55 propyleneglycol oleate and PEG-18 glyceryl oleate/cocoate known with the trade names Antil^{R} 141 and 171, respectively and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature.

Viscosity of the non-cleansing conditioning composition may not be more than 100,000 mPa.s at 20°C measured with Brookfield Rheometer at a shear rate of 10 sec⁻¹.

It should especially be noted that the effects of the inventive compositions become more and more visible after repeated usage. Furthermore, it has also been found out that the effects are much more pronounced when compositions used for cleansing and for conditioning both comprises at least one xanthine and its derivative according to the formula above and at least one carboxylic acid according to the formula above. Therefore, a further object of the present invention is a kit comprising at least two compositions, wherein one of the compositions is a cleansing composition based on at least one surfactant and another composition without a cleansing effect wherein bot compositions comprise at least one xanthin and its derivative according to the above general formula and at least one carboxylic acid according to the general formula above.

The following examples are to illustrate the invention, but not to limit. The compositions according to the invention are prepared by mixing the individual components in water, whereby it is also possible to use pre-mixtures of various ingredients.

### Example 1

The following compositions were prepared and tested against each other in a half side test with 10 volunteers. The volunteer's hair was washed with a commercially available shampoo composition and after towel drying compositions below were applied onto hair and without rinsing off hair was dried and hair properties were evaluated by 3 hair dressers by giving marks between 0 and 2 for each side, 0 is no effect, 1 is average effect and 2 is distinguishable effect. The table below includes the total scores of 10 evaluations. It should be noted that for each half side average of 3 hairdressers evaluation is taken into account.

The following compositions were tested as shown in Table I

**Table I: Conditioning compositions tested.**

| | Concentration % by weight | | | |
|---|---|---|---|---|
| | A | B | C | D |
| Cetrimonium chloride | 0.25 | 0.25 | 0.25 | 0.25 |
| Glyoxylic acid | - | 1 | 0.50 | - |
| Caffeine | 0.50 | - | 0.25 | - |
| Ethanol | 16.00 | 16.00 | 16.00 | 16.00 |
| Fragrance, Preservative | q.s. | q.s. | q.s. | q.s. |
| Sodium hydroxide | q.s. to pH 4.7 | | | |
| Water | q.s to 100 | | | |

It should be noted that only the composition C is according to the invention. The test design was set up in order to prove the synergistic effect of the claimed ingredients.

Results of the hairdresser's evaluation are presented in the Table II below.

**Table II: Results of the hairdresser's evaluation.**

| | A vs D | | B vs D | | C vs D | |
|---|---|---|---|---|---|---|
| Property | A | D | B | D | C | D |
| | | | | | | |
| Bounce | 9 | 3 | 6 | 4 | 6 | 10 |
| Smoothness | 11 | 3 | 9 | 4 | 2 | 5 |
| Softness | 13 | 3 | 9 | 4 | 3 | 7 |
| Lightness/Looseness | 7 | 8 | 8 | 3 | 5 | 10 |
| Grip | 3 | 13 | 3 | 6 | 8 | 4 |

From the above results it is clear that only with composition C the hair becomes less soft, less bouncy, less smooth lighter and has more grip. The effect is synergistic since the individual components, caffeine and glyoxylic acid improves the smoothness, bouncy and hair has with less grip.

### Example 2

| | |
|---|---|
| Dimethicone | 25.0 |
| Caffeine | 0.5 |
| Glyoxylic acid | 0.5 |
| Cetrimonium chloride | 0.5 |
| Water | q.s. to 100 |

The above composition is a 2 phase composition which is applied onto hair after homogenously mixing the two phases by shaking. The oil (dimethicone) and water phases separate in a short period of time into two phases.

Above composition is applied onto towel dried hair after shampooing. It may also be applied onto dry hair. It gives hair more grip and shine.

### Example 3

| | |
|---|---|
| Sodium lauryl ether sulfate | 11.0 (% by wt.) |
| Coco glucoside | 4.0 |
| Cocoamidopropyl betaine | 1.5 |
| Cationic polymer (Polyquaternium-1 0) | 0.2 |
| Caffeine | 0.5 |
| Glyoxylic acid | 0.5 |
| Perfume, preservative | q.s |
| PEG-60-hydrogenated castor oil | 0.5 |
| PEG-18 Glyceryl cocoate/oleate | 2.0 |
| Sodium hydroxide | q.s to pH 5.0 |
| Water | q.s. to 100.0 |

The above composition was tested in a half side blind test against a composition not comprising caffeine and glyoxylic acid. 10 volunteers having a shoulder length hair participated to the test and a hairdresser carried out washing using equal amount of shampoo composition for both sides. Afterwards the hair conditioning effect of the shampoo was evaluated by hairdresser.

Following results were observed.

**Table I: Results of half side test. The numbers presents the preference. In case of no preference it should be understood that both sides do not differentiate noticeably.**

| | **Inventive** | **Comparative** | **No preference** |
|---|---|---|---|
| **Wet Hair** | | | |
| Combability | 2 | 5 | 3 |
| Smoothness | 1 | 6 | 3 |
| Roughness | 3 | 0 | 7 |

| **Dry Hair** | | | |
|---|---|---|---|
| Combability | 3 | 5 | 2 |
| Smoothness | 1 | 7 | 2 |
| Roughness | 4 | 0 | 6 |
| Grip | 7 | 1 | 2 |
| Volume | 2 | 7 | 1 |
| Body | 8 | 1 | 1 |
| Shine | 4 | 5 | 1 |

From the above results it is clear that the inventive composition provides hair more grip.

In a further two test series; the shampoo composition comprising only one of the two ingredients was tested against a composition not comprising any of the two ingredients. With the two ingredients caffeine and glyoxylic are meant. The concentration of the compound present in the composition was adjusted to the total concentration of both ingredients. It was observed that the above results could not be obtained with only one of the two ingredients. In both cases it was observed that the inventive composition was providing hair much more grip. This effect is a synergistic effect.

Similar results were observed with the shampoo compositions below.

### Example 4

| | |
|---|---|
| Sodium lauryl ether carboxylate (10EO) | 5.0 (% by wt.) |
| Coco glucoside | 5.0 |
| Cocoamidopropyl betaine | 5.0 |
| Cationic polymer (Polyquaternium-7) | 0.2 |
| Benzophenone-3 | 0.3 |
| Caffeine | 0.5 |
| Glyoxylic acid | 0.5 |
| Perfume, preservative | q.s. |
| PEG-60-hydrogenated castor oil | 0.5 |
| PEG-18 Glyceryl cocoate/oleate | 1.0 |
| Lactic acid | 0.5 |
| Water | q.s. to 100.0 |

The pH of the composition is 4.5.

### Example 5

| | |
|---|---|
| Sodium laureth sulfate | 10.0 |
| Cocoamidopropyl betaine | 3.0 |
| Sodium lauroyl glutamate | 3.0 |
| Decyl glucoside | 3.0 |
| Polyquaternium-37 | 0.5 |
| PEG-3 distearate | 0.8 |
| Sebumol ODPC | 0.2 |
| Caffeine | 0.3 |
| Glyoxylic acid | 0.3 |
| Benzylalcohol | 0.5 |
| Perfume, preservative | q.s. |
| Sodium chloride | 0.80 |
| Citric acid | q.s. to pH 5.2 |
| Water | q.s to 100.0 |

### Example 6

### Foam shampoo

| | |
|---|---|
| Coco glucoside | 5.0 |
| Cocoamidopropyl betaine | 6.0 |
| Sodium laureth sulfate | 4.0 |
| Cationic guar gum* | 0.5 |
| Benzylalcohol | 0.5 |
| Glycoprotein | 0.1 |
| Perfume, preservative | q.s. |
| Caffeine | 0.65 |
| Benzphenone-3 | 0.5 |
| Hempseed oil | 0.5 |
| Glyoxylic acid | 0.65 |
| Water | ad 100.0 |

| | |
|---|---|
| *: Jaguar C13S | |

The pH of the composition is 5.4. The above composition is a very low viscosity composition, in any case a viscosity lower than 500 mPa.s measured at ambient temperature and with Höppler viscosimeter, confectioned into a pump-foamer as purchased from the company Air-Spray - Germany and showed excellent conditioning effect in terms of giving hair more grip.

Similarly and aerosol foam shampoo was prepared by confectioning the above composition at a weight ratio of 90/10 - composition/propellant- using propane-butane mixture as a propellant. The foam shampoo so obtained showed excellent cleansing, conditioning effects in terms of giving hair more grip.

### Example 7

| | |
|---|---|
| Sodium laureth sulfate | 5.0 |
| Cocoamidopropyl betaine | 5.0 |
| Lauroyl glutamate | 3.0 |
| Decyl glucoside | 5.0 |
| Polyquaternium-10 | 0.5 |
| PEG-3 distearate | 0.8 |
| Evening Primerose oil | 0.3 |
| Ethylhexylmethocxycinnamate | 0.3 |
| Basic red 51 | 0.10 |
| Ubichinone | 0.10 |
| Benzylalcohol | 0.5 |
| Caffeine | 0.5 |
| Perfume, preservative | q.s. |
| Sodium chloride | 0.80 |
| Glyoxylic acid | 0.5 |
| Water | ad 100.0 |

The pH of the composition is 5.2

Above shampoo showed excellent conditioning effect in terms of more grip and body and also delivered a light red shine onto hair. Protection effect against UV light was well pronounced.

### Example 8

| | |
|---|---|
| Cetylstearylalcohol | 5.0 (% by weight) |
| Stearyltrimethylammoniumchlorid | 1.0 |
| Stearamidopropyldimethylamine | 1.0 |
| Benzylalcohol | 2.5 |
| Polysilicone-15 | 0.8 |
| Passiflora incarnata seed oil | 1.0 |
| Caffeine | 0.4 |
| Glyoxylic acid | 0.4 |
| Fragrance, preservative | q.s. |
| Citric acid | 0.8 |
| Wasser | ad 100.0 |

The pH of the composition is 3.0.

Above composition was tested in a half side test against compositions not comprising caffeine and glyoxylic acid. The test was carried out with 10 female volunteers having shoulder length hair. Volunteers' hair was washed with a conventional shampoo composition and towel dried and the equal amount of above composition and the comparative without the active ingredients were applied to each side and processed for 5 min. Afterwards the hair was rinsed off and evaluated by a hair dresser for its conditioning properties.

Following results were observed.

**Table II: Results of half side test. The numbers presents the preference. In case of no preference it should be understood that both sides do not differentiate noticeably.**

| | **Inventive** | **Comparative** | **No preference** |
|---|---|---|---|
| **Wet Hair** | | | |
| Combability | 3 | 6 | 1 |
| Smoothness | 1 | 7 | 2 |
| Roughness | 5 | 2 | 3 |

| **Dry Hair** | | | |
|---|---|---|---|
| Combability | 2 | 6 | 2 |
| Smoothness | 2 | 7 | 1 |
| Roughness | 5 | 1 | 4 |
| Grip | 8 | 1 | 1 |
| Volume | 2 | 7 | 1 |
| Body | 8 | 1 | 1 |
| Shine | 3 | 5 | 2 |

From the above results it is clear that the inventive composition improves grip, volume and body of hair.

In a further two test series; the conditioner composition comprising only one of the two ingredients was tested against a composition not comprising any of the two ingredients. With the two ingredients caffeine and glyoxylic are meant. The concentration of the compound present in the composition was adjusted to the total concentration of both ingredients. It was observed that the above results could not be obtained with only one of the two ingredients. In both cases it was observed that the inventive composition was conditioning hair in a much better way in terms of grip and body and this effect is a synergistic effect.

At the same time protection effect against UV exposure was also confirmed with the same method as it was done in Example 1.

Similar results were observed with conditioner compositions below.

### Example 9

### Foam conditioner

| | |
|---|---|
| Quaternium-80 | 0.2 (Gew.-%) |
| Polyquaternium-11 | 0.7 |
| PEG-60-hydrogenated ricinus oil | 0.5 |
| Fragrance, preservative | q.s. |
| Polysilicone-15 | 0.1 |
| Guarana extract | 0.1 |
| Passiflora incarnata seed oil | 0.1 |
| Glyoxylic acid | 0.7 |
| Caffeine | 0.7 |
| Wasser | ad 100.0 |

pH of the composition is adjusted to 3.4. The composition is suitable for leave-in and rinse off. In leave-in application, amount (whole composition as a conditioner applied onto hair) used is obviously less than in the case of a rinse of application. The composition is packed into an aerosol can with 90/10 ratio, by weight, liquid composition to propellant. As propellant propane, butane mixture is used.

10 female volunteer were asked to use the above composition at home after washing their hair with their usual shampoo and using the above composition as leave in conditioner by applying on towel dry hair. The comments after a 3 week usage period were
- my hair feels with more grip,
- I have more hair when I touch my hair,
- My hair does not move any more every time when I move my head,
- My hair has less volume
- I can still comb my hair very easily

Similar results were obtained with the following examples.

### Example 10

### Conditioner

| | |
|---|---|
| Cetylstearylalcohol | 5.0 (% by weight) |
| Cetrimoniumchloride | 1.0 |
| Panthenol | 0,4 |
| Dimethicone | 0.75 |
| Hydroxypropyl Guar Hydroxypropyltrimonium | |
| Chloride | 1.0 |
| Polysilicone-15 | 1.0 |
| Caffeine | 0.5 |
| Glyoxylic acid | 0.5 |
| Menthol | 0.3 |
| Fragrance, preservative | q.s. |
| Citric acid | 0.3 |
| Wasser | ad 100.0 |

The pH of the composition is 3.6.

### Example 11

### Conditioner

| | |
|---|---|
| Cetylstearylalcohol | 10.0 (% by weight) |
| Dioleoylethyldimethylammonium ethosulfate | 0.5 |
| Ceteareth 20 | 3.0 |
| Panthenol | 0.4 |
| Phenyltrimethicone | 0.25 |
| Hydroxypropyl Guar Hydroxypropyltrimonium | |
| Chloride | 0.75 |
| Polysilicone-15 | 0.5 |
| Caffeine | 0.15 |
| Glyoxylic acid | 0.15 |
| Basic red 51 | 0.10 |
| Basic red 76 | 0.15 |
| Fragrance, preservative | q.s. |
| citric acid | 0.5 |
| Wasser | ad 100.0 |

The pH of the composition is 3.4.

The above composition gave in addition to improved grip and body, excellent red shimmer on medium blonde hair.

### Example 12

| | |
|---|---|
| Alcohol | 35.0 |
| Avena sativa extract | 0.5 |
| Caffeine | 0.5 |
| Glyoxylic acid | 0.5 |
| Menthol | 0.3 |
| Cetrimonium chloride | 1.0 |
| Water | q.s. to 100 |

The pH of the above composition is 3.8.

The above composition is possible to apply onto wet or dry hair from a pump spray equipped with a mechanical spray head. It is also possible to apply from a pressurized aerosol container.

## Claims

1. Composition for hair **characterized in that**, it comprises at least one xanthine or its derivative according to the general formula 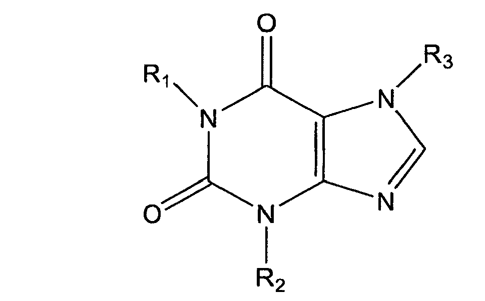 wherein R₁, R₂ and R₃ are independent from each other H, or substituted or unsubstituted C₁ to C₅ alkyl at least one carboxylic acid selected from malonic acid and glyoxylic acid and at least one conditioning compound selected from oily substances, nonionic substances, cationic amphiphilic ingredients, cationic polymers or their mixture.

2. Composition according to claim 1 **characterized in that** it comprises at least one xanthine or its derivative at a concentration of 0.001 to 5% by weight and at least one carboxylic acid at a concentration of 0.001 to 5% by weight, calculated to total composition, preferably at a weight ratio of 10:1 to 1:10.

3. Composition according to claims 1 and 2 **characterized in that** at least one xanthine or its derivative is selected from xanthine, caffeine, theobromine and theophylline.

4. Composition according to any of the preceding claims **characterized in that** it comprises caffeine and glyoxylic acid.

5. Composition according to any of the preceding claims **characterized in that** oily substances are selected from silicone oils, synthetic oils, natural oils and mineral oils.

6. Composition according to any of the preceding claims **characterized in that** it comprises as a conditioning compound a cationic polymer and/or a cationic and/or a cationizable surfactant according to general formula
R₉- A R₁₀- B
wherein R₉ is a saturated or unsaturated, straight or branched alkyl group with 8 to 24 C atoms, R₁₀ is a straight or branched alkyl group with 1 to 4 C atoms, A is a group selected from O, 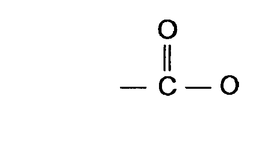 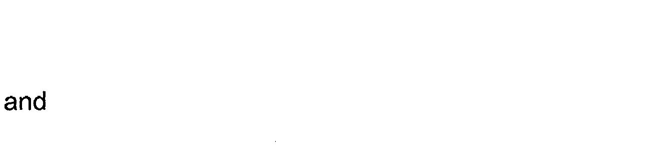 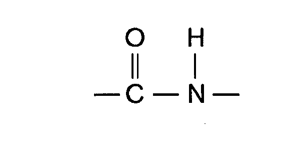 and B is selected from 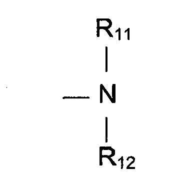 wherein R₁₁ and R₁₂ are the same or different and are H or an alkyl with 1 to 4 C atoms, hydroxyl alkyl with 1 to 4 C atoms and di hydroxyl alkyl with 2 to 4 C atoms, 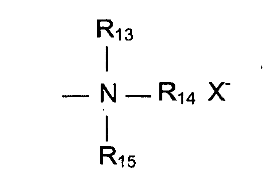 R₁₃ and R₁₅ are the same or different, an alkyl with 1 to 4 C atoms, hydroxyl alkyl with 1 to 4 C atoms and di hydroxyl alkyl with 2 to 4 C atoms, R₁₄ is an alkyl with 1 to 4 C atoms, hydroxyl alkyl with 1 to 4 C atoms or di hydroxyl alkyl with 2 to 4 C atoms and
- R₁₀- A -R₉
wherein R₉, A and R₁₀ have the above meaning and X is chloride, bromide, methosulfate,
or
a quaternary ammonium surfactant according to the general formula 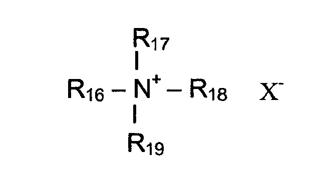 where R₁₆ is a saturated or unsaturated, branched or non-branched alkyl chain with 8-24 C atoms and R₁₇ is unsaturated or saturated, branched or non-branched alkyl chain with 1 - 24 C atoms and R₁₈ and R₁₉ are lower alkyl chain with 1 to 4 carbon atoms which may be substituted with one or more hydroxyl groups, and X is anion such as chloride, bromide, methosulfate.

7. Composition according any of the preceding claims **characterized in that** it comprises at least one UV filter and/or at least one polyol and/or natural plant extract and/or at least one sequestering agent and/or at least one flavonoid and/or at least one pyrrolidone carboxylic acid or its salts or its esters with an alkyl chain and/or at least one ceramide,

8. Composition according to any of the preceding claims **characterized in that** it comprises at least one direct dye, preferably a cationic direct dye.

9. Composition according any of the preceding claims **characterized in that** it comprises at least one surfactant selected from anionic, nonionic and amphoteric ones.

10. Composition according claim 9 **characterized in that** it comprises at least one anionic surfactant, at least one nonionic surfactant and at least one amphoteric surfactant.

11. Composition according any of the preceding claims **characterized in that** it comprises at least one fatty alcohol according to the formula
R₁₄-OH
wherein R₁₄ is a saturated or unsaturated, branched or non-branched alkyl chain with 8 - 24 C atoms.

12. Use of composition according to any of the preceding claims for conditioning hair, preferably for improving rigidity and body of hair.

## Patentansprüche

1. Zusammensetzung für Haare, **dadurch gekennzeichnet, dass** sie mindestens ein Xanthin oder dessen Derivat gemäß der allgemeinen Formel 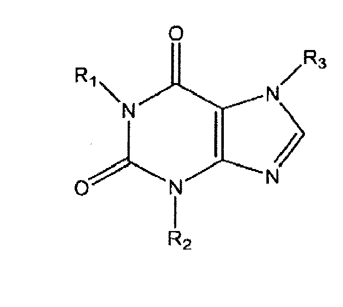 aufweist,
wobei R₁, R₂ und R₃ unabhängig voneinander H oder eine substituierte oder unsubstituierte C₁₋₅-Alkylgruppe, mindestens eine Carbonsäure, ausgewählt aus Malonsäure und Glyoxylsäure, oder mindestens eine konditionierende Verbindung sind, ausgewählt aus öligen Substanzen, nichtionischen Substanzen, kationischen amphiphilen Inhaltsstoffen, kationischen Polymeren oder Gemischen derselben.

2. Zusammensetzung nach Anspruch 1, **dadurch gekennzeichnet, dass** sie mindestens ein Xanthin oder dessen Derivat in einer Konzentration von 0,001 Gewichts-% bis 5 Gewichts-% und mindestens eine Carbonsäure in einer Konzentration von 0,001 Gewichts-% bis 5 Gewichts-%, bezogen auf die Gesamtzusammensetzung, aufweist, vorzugsweise in einem Gewichtsverhältnis von 10:1 bis 1:10.

3. Zusammensetzung nach Anspruch 1 und 2, **dadurch gekennzeichnet, dass** mindestens ein Xanthin oder dessen Derivat aus Xanthin, Coffein, Theobromin und Theophyllin ausgewählt ist.

4. Zusammensetzung nach einem der vorhergehenden Ansprüche, **dadurch gekennzeichnet, dass** sie Coffein und Glyoxylsäure aufweist.

5. Zusammensetzung nach einem der vorhergehenden Ansprüche, **dadurch gekennzeichnet, dass** ölige Substanzen aus Silikonölen, künstlichen Ölen, natürlichen Ölen und Mineralölen ausgewählt sind.

6. Zusammensetzung nach einem der vorhergehenden Ansprüche, **dadurch gekennzeichnet, dass** sie als eine konditionierende Verbindung ein kationisches Polymer und/oder ein kationisches und/oder ein kationisierbares Tensid gemäß der allgemeinen Formel
R₉——A——R₁₀—— B
wobei R₉ eine gesättigte oder ungesättigte, geradkettige oder verzweigte Alkylgruppe mit 8 bis 24 C-Atomen ist, R₁₀ eine geradkettige oder verzweigte Alkylgruppe mit 1 bis 4 C-Atomen ist, A eine Gruppe ist, ausgewählt aus O, 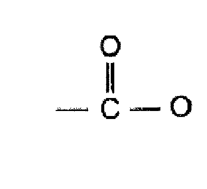 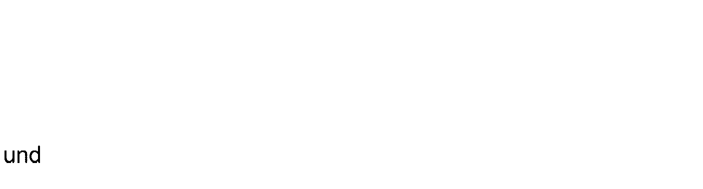 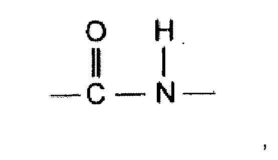 und B ausgewählt ist aus 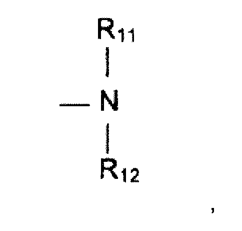 wobei R₁₁ und R₁₂ gleich oder verschieden sind und H oder eine Alkylgruppe mit 1 bis 4 C-Atomen, eine Hydroxyalkylgruppe mit 1 bis 4 C-Atomen und eine Dihydroxyalkylgruppe mit 2 bis 4 C-Atomen sind, 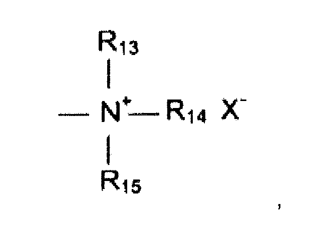 wobei R₁₃ und R₁₄ gleich oder verschieden sind, eine Alkylgruppe mit 1 bis 4 C-Atomen, eine Hydroxyalkylgruppe mit 1 bis 4 C-Atomen und eine Dihydroxyalkylgruppe mit 2 bis 4 C-Atomen sind, R₁₄ eine Alkylgruppe mit 1 bis 4 C-Atomen, eine Hydroxyalkylgruppe mit 1 bis 4 C-Atomen oder eine Dihydroxyalkylgruppe mit 2 bis 4 C-Atomen ist, und
—R₁₀—A——R₉
wobei R₉, A und R₁₀ die obige Bedeutung haben, und X Chlorid, Bromid, Methosulfat ist,
oder
ein quartäres Ammoniumtensid gemäß der allgemeinen Formel 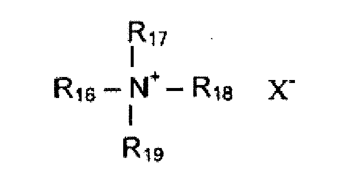 aufweist, wobei R₁₆ eine gesättigte oder ungesättigte, verzweigte oder unverzweigte Alkylkette mit 8 bis 24 C-Atomen ist und R₁₇ eine ungesättigte oder gesättigte, verzweigte oder unverzweigte Alkylkette mit 1 bis 24 C-Atomen ist und R₁₈ und R₁₉ niedere Alkylketten mit 1 bis 4 Kohlenstoffatomen sind, welche mit einer oder mehreren Hydroxygruppen substituiert sein können, und X ein Anion wie z.B. Chlorid, Bromid, Methosulfat ist.

7. Zusammensetzung nach einem der vorhergehenden Ansprüche, **dadurch gekennzeichnet, dass** sie mindestens einen UV-Filter und/oder mindestens ein Polyol und/oder natürlichen Pflanzenextrakt und/oder mindestens ein Maskierungsmittel und/oder mindestens ein Flavonoid und/oder mindestens eine Pyrrolidoncarbonsäure oder deren Salze oder deren Ester mit einer Alkylkette und/oder mindestens ein Ceramid aufweist.

8. Zusammensetzung nach einem der vorhergehenden Ansprüche, **dadurch gekennzeichnet, dass** sie mindestens einen Direktfarbstoff, vorzugsweise einen kationischen Direktfarbstoff, aufweist.

9. Zusammensetzung nach einem der vorhergehenden Ansprüche, **dadurch gekennzeichnet, dass** sie mindestens ein Tensid aufweist, ausgewählt aus anionischen, nichtionischen und amphoteren.

10. Zusammensetzung nach Anspruch 9, **dadurch gekennzeichnet, dass** sie mindestens ein anionisches Tensid, mindestens ein nichtionisches Tensid und mindestens ein amphoteres Tensid aufweist.

11. Zusammensetzung nach einem der vorhergehenden Ansprüche, **dadurch gekennzeichnet, dass** sie mindestens einen Fettalkohol gemäß der Formel
R₁₄-OH
aufweist, wobei R₁₄ eine gesättigte oder ungesättigte, verzweigte oder unverzweigte Alkylkette mit 8 bis 24 C-Atomen ist.

12. Verwendung einer Zusammensetzung nach einem der vorhergehenden Ansprüche zum Konditionieren von Haaren, vorzugsweise zum Verbessern der Festigkeit und der Struktur von Haaren.

## Revendications

1. Composition pour cheveux, **caractérisée en ce qu'**elle comprend au moins une xanthine, ou son dérivé, selon la formule générale 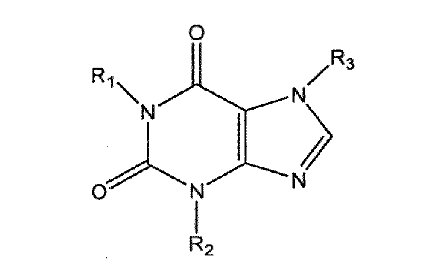 dans laquelle R₁, R₂ et R₃ sont indépendamment les uns des autres des atomes H, ou des groupes alkyles en C₁ à C₅ substitués ou non substitués, au moins un acide carboxylique choisi parmi l'acide malonique ou l'acide glyoxylique et au moins un composé de traitement choisi parmi des substances à base d'huiles, des substances non ioniques, des constituants amphiphiles cationiques, des polymères cationiques ou des mélanges de ceux-ci.

2. Composition selon la revendication 1, **caractérisée en ce qu'**elle comprend au moins une xanthine, ou son dérivé, à une concentration de 0,001 à 5 % en poids et au moins un acide carboxylique à une concentration de 0,001 à 5 % en poids, calculées par rapport à la composition totale, de préférence dans un ratio en poids de10:1à1:10.

3. Composition selon les revendications 1 et 2, **caractérisée en ce qu'**au moins une xanthine, ou son dérivé, sont choisis parmi la xanthine, la caféine, la théobromine et la théophylline.

4. Composition selon l'une quelconque des revendications précédentes, elle comprend de la caféine et de l'acide glyoxylique.

5. Composition selon l'une quelconque des revendications précédentes, **caractérisée en ce que** des substances à base d'huiles sont choisies parmi des huiles silicones, des huiles synthétiques, des huiles naturelles et des huiles minérales.

6. Composition selon l'une quelconque des revendications précédentes, **caractérisée en ce qu'**elle comprend, en tant que composé de traitement, un polymère cationique, et/ou un tensioactif cationique, et/ou un tensioactif pouvant être rendu cationique, selon la formule générale
R₉—— A —— R₁₀—— B
dans laquelle R₉ est un groupe alkyle saturé ou insaturé, linéaire ou ramifié, avec 8 à 24 atomes de C, R₁₀ est un groupe alkyle linéaire ou ramifié avec 1 à 4 atomes de C, A est un groupe choisi parmi un atome O, un groupe 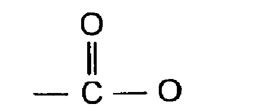 et un groupe 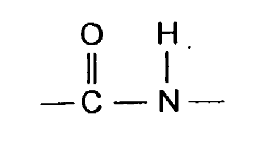 et B est choisi parmi un groupe 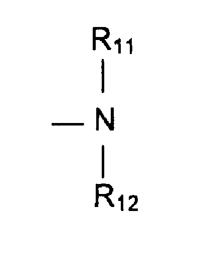 dans lequel R₁₁ et R₁₂ sont identiques ou différents et sont des atomes H ou des groupes alkyles avec 1 à 4 atomes de C, des groupes hydroxy-alkyles avec 1 à 4 atomes de C et des groupes dihydroxy-alkyles avec 2 à 4 atomes de C,
un groupe 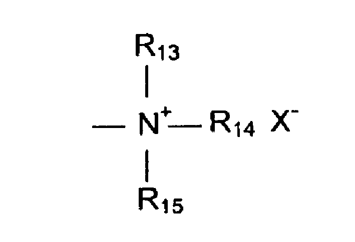 R₁₃ et R₁₅ sont identiques ou différents, et représentent un groupe alkyle avec 1 à 4 atomes de C, un groupe hydroxy-alkyle avec 1 à 4 atomes de C et un groupe dihydroxy-alkyle avec 2 à 4 atomes de C, R₁₄ est un groupe alkyle avec 1 à 4 atomes de C, un groupe hydroxy-alkyle avec 1 à 4 atomes de C ou un groupe dihydroxy-alkyle avec 2 à 4 atomes de C et un groupe
—R₁₀—A——R₉
dans lequel R₉, A et R₁₀ ont les significations décrites ci-dessus et où X est un anion chlorure, bromure, méthosulfate,
ou
un tensioactif ammonium quaternaire selon la formule générale 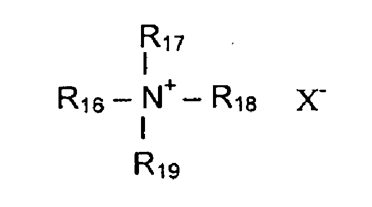 où R₁₆ est une chaine alkyle saturée ou insaturée, ramifiée ou linéaire avec 8 à 24 atomes de C et R₁₇ est une chaine alkyle insaturée ou saturée, ramifiée ou linéaire avec 1 à 24 atomes de C, et R₁₈ et R₁₉ sont une chaine alkyle de plus faible poids moléculaire avec 1 à 4 atomes de C qui peut être substituée par un ou plusieurs groupes hydroxyles, et X est un anion tel que le chlorure, le bromure, le méthosulfate.

7. Composition selon l'une quelconque des revendications précédentes, **caractérisée en ce qu'**elle comprend au moins un filtre UV, et/ou au moins un polyol, et/ou un extrait naturel de plante, et/ou au moins un agent séquestrant, et/ou au moins un flavonoïde, et/ou au moins un acide pyrrolidone-carboxylique, ou des sels de celui-ci, ou des esters de celui-ci, avec une chaine alkyle et/ou au moins un céramide.

8. Composition selon l'une quelconque des revendications précédentes, **caractérisée en ce qu'**elle comprend au moins un colorant direct, de préférence un colorant direct cationique.

9. Composition selon l'une quelconque des revendications précédentes, **caractérisée en ce qu'**elle comprend au moins un tensioactif choisi parmi les tensioactifs anioniques, non ioniques et amphotères.

10. Composition selon la revendication 9, **caractérisée en ce qu'**elle comprend au moins un tensioactif anionique, au moins un tensioactif non ionique et au moins un tensioactif amphotère.

11. Composition selon l'une quelconque des revendications précédentes, **caractérisée en ce qu'**elle comprend au moins un alcool gras selon la formule
R₁₄-OH
dans laquelle R₁₄ est une chaine alkyle saturée ou insaturée, ramifiée ou linéaire avec 8 à 14 atomes de C.

12. Utilisation de la composition selon l'une quelconque des revendications précédentes pour le traitement des cheveux, de préférence pour améliorer la rigidité et la structure des cheveux.
